# EUROPEAN PATENT APPLICATION

(11) **EP 2 388 237 A1**
(43) Date of publication of application: **23.11.2011**
(21) Application number: 09834157.1
(22) Date of filing: 27.11.2009
(51) Int. Cl.: C01B 33/40, C01F 7/00, B01J 23/00, B01J 20/12, B01D 53/02

(54) **MICRO- AND NANO-STRUCTURED COMPOSITE MATERIALS BASED ON LAMINAR DOUBLE HYDROXIDES OF HYDROTALCITE TYPE AND SILICATES OF THE CLAY FAMILY**

(30) Priority: 22.12.2008 ES 200803642
(71) Applicant: Consejo Superior de Investigaciones Cientificas (CSIC), 28006 Madrid (ES)
(72) Inventor: RUIZ HITZKY, Eduardo, E-28049 Madrid (ES); ARANDA GALLEGO, Pilar, E-28049 Madrid (ES); GÓMEZ AVILÉS, Almudena, E-28049 Madrid (ES)
(74) Representative: Pons Ariño, Angel
(86) International application number: PCT/ES2009/070535
(87) International publication number: WO 2010/072870

(57) **Abstract**

The present invention relates to micro- or nano-structured composite materials based on layered double hydroxides of hydrotalcite type and silicates of the clay family. The invention also relates to the preparation process of these materials as well as to their use in various applications such as adsorbent, both of gases and of contaminants in aqueous medium, absorbent, acid neutralizer, ion exchanger, in medical and biological applications, as supports of materials of biological origin such as enzymes, as charges in polymers, as well as precursors of metal oxides and of catalysts.

## Description

### Field of the invention

The present invention relates to micro- or nano-structured composite materials based on layered double hydroxides of hydrotalcite type and silicates of the clay family. Therefore, the invention is within the sector of novel materials, whilst its applications are mainly located in the chemical (adsorbent, acid neutralizer, ion exchanger, catalyst), pharmaceutical and medical (additives, active principles) and environmental sectors (water, adsorption of contaminating gases), as well as protector against corrosion.

### State of the art

Layered double hydroxides (LDHs) of hydrotalcite type are also known as "anionic clays" due to their anion exchange properties (inversely to what occurs with clays which are cation exchange materials). The structure of the LDHs corresponds to that of the natural mineral called hydrotalcite, Mg₆Al₂(OH)₁₆CO₃4H₂O, which is usually described from the structure of the brucite of composition Mg(OH)₂, where the M²⁺ ions are partially substituted by M³⁺ ions, which creates a charge deficit in the layers which is compensated by anions placed in the interlayer space and which may be exchanged by ion exchange processes. In general LDHs are usually described by the general formula [M²⁺₁₋ₓ M³⁺ₓ(OH)2] [Aⁿ⁻_{x/n}·zH₂O] wherein M²⁺ (normally) and M (III) are metal ions and Aⁿ⁻ is the anion which compensates the deficit of negative charge in the layers and may be of an inorganic cation (typically Cl⁻, NO³⁻ CO₃²⁻, SO₄²⁻) or organic compounds of highly varied nature provided with carboxylate, sulfonate, groups, etc. The notation M²⁺ may indicate the presence of more than one type of divalent cation and M³⁺ of more than one trivalent cation, however, the molar ratio x = M³⁺/ (M³⁺+ M²⁺) must remain confined between 0.2 and 0.4. LDHs may be prepared by a combination of a wide variety of metal ions M²⁺ such as, for example, Mg²⁺, Zn²⁺, Fe²⁺, Cu²⁺, Ca²⁺, Mn²⁺, Ni²⁺ or CO²⁺ and of M³⁺ metal ions, for example Al³⁺, Cr³⁺, Mn³⁺, Fe³⁺ or Ga³⁺ among other possible combinations (A. de Roy, C. Forano, K. EI Malki and J.-P. Besse In: M.L. Ocelli and H. Robson, Editors, Expanded Clays and Other Microporous Solids, VNR, New York (1992), p. 108.). The most typical LDHs preparation method is the socalled co-precipitation which, with slight variants, consists of the precipitation at a controlled pH of the mixed hydroxide from dissolved salts of the metal cations, in the presence of the anion which one wants to incorporate in the interlayer space. It is also possible to form LDHs by a process known as "reconstruction", from the respective oxides, when they have been formed from a LDH calcined at temperatures of between 500-80ºC, which is then rehydrated in the presence of anions and water.

LDHs have interest in various areas of application, including their use as polymer charge, as substrate for chromatography, as antacid, as support or agent for the controlled release of drugs and pesticides, as substrate for the immobilization of enzymes, as precursors of magnetic materials or of catalysts, as adsorbents of dyes, scavenger of contaminating species, fluorides, acids and gases such as carbon dioxide and sulphur and nitrogen oxides, as well as protection against corrosion (F. Li, X. Duan, Applications of layered double hydroxides. Struct. Bonding (Berlin) 2006, 119, 193; F. Zhang, M. Sun, S. Xu, L. Zhao, B. Zhang. Fabrication of oriented layered double hydroxide films by spin coating and their use in corrosion protection, Chemical Engineering Journal, 141 (2008) 362-367).

The preparation of LDHs supported on other inorganic solids has a twofold interest. On the one hand, the association with the solid can facilitate the separation process of the colloids formed in the synthesis. On the other hand, the preparation on a support makes it possible to obtain materials of greater specific surface, which may have advantages in many of the applications for which they are prepared. In relation with this subject, the synthesis of LDHs of magnesium and aluminium has been described, supported on carbon nanofibers, for their use as catalysts in the synthesis of methyl-isobutyl ketone (MIBK) (F.Winter, V. Koot, A. Jos van Dillen, J. W. Geus, K. P. de Jong, Hydrotalcites supported on carbon nanofibers as solid base catalysts for the synthesis of MIBK, Journal of Catalysis, 236 (2005) 91-100). Composites of LDHs of magnesium and aluminium have also been described with zeolite Y (Hydrotalcite-zeolite composites and catalysts thereof by NOx storage method, Patent WO 2008/066275-A1). Another type of already prepared composites relates to the use of cellulose as support for LDHs for their use in the adsorption of fluorides in aqueous solutions (S. Mandal, S. Mayadevi, Cellulose supported layered double hydroxides for the adsorption of fluoride from aqueous solution, Chemosphere 72 (2008) 995-998).

There is no record of the preparation of composite materials comprised of LDHs and clays combined on a micro or nanometric scale. The use of clays as substrates for the growth of LDHs has an undoubted interest since clays have characteristics complementary to the LDHs, such as their properties of cation exchange or the possibility of presenting different textural properties, of adsorption, ion exchange and chemical reactivity. In this sense, it is of particular relevance to indicate the fact that clays, compounds described as hydrated aluminosilicates of natural or synthetic origin, generally have a layered structure with very anisotropic particles, as is the case of the smectites among which the montmorillonites are included. Clays have been widely used by man as raw material for the preparation of ceramics, in construction and civil engineering, as additive of plastics, paints, paper, rubber, in cosmetic and medicine, in food, as adsorbent, catalyst support, etc. In addition to layered clays, there are clays of fibrous nature such as sepiolite and palygorskite (also known as atapulgite), **characterized in that** they have a high specific surface, are highly microporous and have a high number of superficial silanols, which gives it very specific surface properties (E. Ruiz-Hitzky, Molecular access to intracrystalline tunnels of sepiolite, Journal of Materials Chemistry, 11 (2001) 86-91). These superficial properties make them especially interesting as support of nanoparticles of various nature, such as metals, oxides, etc. fundamentally for applications in catalysis (P. Aranda, R. Kun, M.A. Martin-Luengo, S. Letaïef, I. Dékány, E. Ruiz-Hitzky, "Titania-sepiolite nanocomposites prepared by a surfactant templating colloidal route", Chem. Mater. 20, 84-89 (2008)). The formation of LDHs supported on clays is therefore considered an advance in the development of novel micro- and nano-structured composite materials with properties of interest in adsorption processes, ion exchange, basic reserve against acids, rheological properties, etc.

### Brief description of the invention

The present invention is based on three fundamental aspects:
A first aspect of the present invention is the micro- and nano-structured composite, hereinafter composite of the invention, which comprises a silicate of the clay family and a layered double hydroxide of hydrotalcite type.
A second aspect of the present invention is the preparation process of the composite of the invention which comprises the in situ formation of the layered double hydroxide in the presence of a clay.
A third aspect of the present invention is the use of the composite of the invention in various applications such as adsorbent or absorbent, both of gases and of contaminants in aqueous medium, acid neutralizer, ion exchanger, in medical and biological applications, as fillers in polymers, in the protection of corrosion, as well as precursors of metal oxides and of catalysts.

### Detailed description of the invention

The present invention is based on a novel type of composite material, prepared by the inventors, which comprises a silicate of the clay family, hereinafter clay, and a layered double hydroxide of hydrotalcite type, hereinafter LDH. This type of materials are micro- or nano-structured systems wherein the LDH particles are associated to clay particles which can be considered as support of such hydroxides, which are formed in the presence of such clay.

In the present invention silicate of the clay family is understood to be those silicates of aluminium and/or magnesium, natural or synthetic, both of layered structure (montmorillonite, saponite, vermiculite, hectorite, etc), and of fibrous type (sepiolite or palygorskite, also called atapulgite).

The synergy between both components, i.e. between the LDH and the clay, determines properties of interest in processes of adsorption, ion exchange, basic reserve against acids, rheological properties, etc. Indeed, the presence of clay may contribute to the LDH being deposited on a solid which provides a greater specific surface and porosity to the unit. Another advantage relates to the recovery process of the LDHs after their synthesis, since frequently in the preparation of the hydroxides are formed colloids that are difficult to recover, but as they are associated to clay particles it improves their sedimentation or filtration properties. Another advantage relates to the percolation capacity procured by the clay.

An aspect of the present invention is the micro- and nano-structured composite, hereinafter composite of the invention, which comprises a LDH and a clay.

A preferred aspect of the present invention is the composite of the invention wherein the clay is of fibrous morphology.

A more preferred aspect of the present invention is the composite wherein the clay of fibrous morphology is sepiolite.

Another more preferred aspect of the present invention is the composite of the invention wherein the clay of fibrous morphology is palygorskite.

Another preferred aspect of the present invention is the composite of the invention wherein the clay is of layered structure.

Another more preferred aspect of the present invention is the composite of the invention wherein the clay of layered structure is of natural origin, such as smectite or vermiculite clays.

A particular embodiment of the present invention is the composite of the invention wherein the layered clay of natural origin is a smectite clay.

A particular example of the present invention is the composite of the invention wherein the smectite clay is a montmorillonite.

Other particular examples of the present invention is the composite of the invention wherein the smectite clay is a saponite, hectorite or stevensite.

Another more preferred aspect of the present invention is the composite of the invention wherein the layered clay is synthetic.

Another preferred embodiment of the present invention is the composite of the invention wherein the synthetic layered clay is of the hectorite type, such as, for example, Laponite^{®}.

The composite of the invention is obtained by the co-precipitation of the LDH in the presence of the clay, which acts as a support of the LDH or as the inorganic matrix of the composite material. In this way, the formation of LDH particles is produced *in situ* on the clay surface, and interactions may take place between hydroxyl groups of the clay with those of LDH.

Therefore, another aspect of the present invention is the preparation process of the composite of the invention, hereinafter process of the invention, which comprises the in situ formation of the layered double hydroxide in the presence of a clay.

Some of the preparation conditions, such as, for example, synthesis temperature or pH vary depending on the type of LDH used. The LDH may be formed by different pairs of metal ions such as Mg/Al, Zn/Al, Co/Al, Cu/Al, Ni/Al, etc. Homogenous suspensions are obtained of different proportions of LDH/clay which may be aged, centrifuged, washed and consolidated, thus obtaining the LDH-clay composite.

Another preferred aspect of the present invention is the process of the invention wherein the formation of the layered double hydroxide is carried out by co-precipitation.

Another preferred aspect of the present invention is the process of the invention wherein the preparation of the composite involves a hydrothermal treatment.

Another preferred aspect of the present invention is the process of the invention wherein the preparation of the composite involves a conventional thermal treatment.

Another preferred aspect of the present invention is the process of the invention wherein the preparation of the composite involves a treatment by microwave irradiation.

These composites may also be subjected to thermal treatments to obtain novel composite materials formed by the mixed oxides from LDH (layered double oxides, LDO) supported on the clay.

The composite of the invention may have interest in various applications such as additives and fillers of polymers, in food or pharmacology; in chromatography, and in the field of biosensors; as precursors in magnetic materials; in biology and medicine; in catalysis; in protection against corrosion and in environmental protection.

Another aspect of the present invention is the use of the composite of the invention as adsorbent, i.e. as a material capable of trapping or retaining atomic, molecular or polymeric species in its surface or as absorbent, i.e. as a material capable of incorporating those different species in its volume.

Another preferred aspect of the present invention is the use of the composite of the invention as gas adsorbent.

Another preferred aspect of the present invention is the use of the composite of the invention as absorbent of contaminants in aqueous media.

Another aspect of the present invention is the use of the composite of the invention as acid neutralizer.

Another aspect of the present invention is the use of the composite of the invention as ion exchanger.

Another aspect of the present invention is the use of the composite of the invention as active principle in medical and biological applications.

Another aspect of the present invention is the use of the composite of the invention as antacid agent.

Another aspect of the present invention is the use of the composite of the invention as fillers of PVC and other polymers.

Another aspect of the present invention is the use of the composite of the invention as precursors of metal oxides.

Another aspect of the present invention is the use of the composite of the invention as protector against corrosion.

Another aspect of the present invention is the use of the composite of the invention as precursors of catalysts.

Another aspect of the present invention is the use of the composite of the invention as precursors of adsorbents and absorbents.

### Examples of embodiment of the invention

### Example 1. - Preparation of composites which comprise the LDH of formula [Mg₂Al(OH)₆]Cl·xH₂O and sepiolite.

Composites have been prepared of the LDH of formula [Mg₂Al(OH)₆]Cl·xH2O and sepiolite varying the quantities of precursor reagents of the LDH in order to allow the formation of materials whose proportions by weight of LDHaepiolite may vary theoretically from 0.3:1 until 1:1. For this, a solution A is prepared which contains the ions Al³⁺ and Mg²⁺ in stoichiometric proportions to give the indicated LDH. Typically, for a theoretical proportion of 0.3:1 LDH-sepiolite 0.340 g of AlCl₃.6H₂O and 0.573 g of MgCl₂.6H₂O are used in 500 ml of bidistilled water, which is decarbonated, passing through it a stream of N₂. On the other hand, a suspension B is prepared containing 1 g of sepiolite (PANEL® S9 supplied by Tolsa SA) in 100 ml of bidistilled water, wherethrough is also made to pass a stream of N₂. Then solution A is added slowly to B by the use of a peristaltic pump (speed 2 ml/min) maintaining solution B with constant stirring at a pH of 9.0 ± 0.1 by the controlled addition of 1M NaOH. When all solution A has been incorporated in the system, the bubbling and stirring are continued for 4 hours to guarantee an aging of the system which allows the growth of the LDH crystals. After this time period the solid is recovered by centrifugation (3600 rpm during 15 minutes) and it is washed several times with bidistilled water. The solid is later subjected to a controlled thermal treatment with a ramp heating of 26ºC/h until reaching 150ºC, the temperature at which the system is maintained for a further 3 hours. The solid obtained is subjected to a grinding process for its physicochemical characterization by X-ray diffraction techniques, Fourier transform infrared spectroscopy, differential thermal and thermogravimetric analysis, specific surface measurements and porosity, elemental chemical analysis, high resolution nuclear magnetic resonance of ²⁹Si, electronic scanning microscopy and electronic transmission microscopy. The LDH formation yields with respect to the theoretical value are in the range of 60-70% for the proportions tested. The X-ray diffraction technique makes it possible to confirm the formation of crystalline particles of LDH on observing diffraction peaks at 0.77, 0.39 and 0.26 nm. The results of nuclear magnetic resonance of ²⁹Si show the disappearance of the Q² signal which appears at chemical displacement values towards -85.0 ppm with respect to the Si signal of the tetramethylsilane, assigned to the presence of silanol groups localized on the external surface of the sepiolite fibres, developing a new Q³ signal which appears at -96.6 ppm and which can be assigned to the formation of a new Si-O bond by condensation of superficial silanols of the sepiolite with hydroxyl groups of the LDH. Via electronic scanning and transmission microscopy it is possible to observe the growth of micro- and nano-particles of LDH on the sepiolite fibres whereto they are associated coating them.

### Example 2. - Preparation of composites which comprise the LDH of formula [Zn₂Al(OH)₆]Cl·xH₂O and sepiolite.

Composites have been prepared of the LDH of formula [Zn₂Al(OH)₆]Cl·xH₂O and sepiolite varying the quantities of precursor reagents of the LDH in order to allow the formation of materials whose proportions by weight of LDH:sepiolite may vary theoretically from 0.1:1 to 1:1. For this, a solution A is prepared containing the ions Al³⁺ and Zn²⁺ in stoichiometric proportions to give the LDH. Typically, for a theoretical proportion of 0.3:1 LDH-sepiolite 0.127 g of AlCl₃.6H₂O and 0.448 g of ZnCl₂.6H₂O are used in 500 ml of bidistilled water, which is decarbonated, passing through it a stream of N₂. On the other hand a suspension B is prepared containing 1 g of sepiolite (PANEL® S9, Tolsa S.A.) in 100 ml of bidistilled water, wherethrough is also made to pass a stream of N₂. Then solution A is added slowly to B by the use of a peristaltic pump (speed 2 ml/min) maintaining solution B with constant stirring at 55 ± 0.5 ºC and a pH of 9.0 ± 0.1 by the controlled addition of 1 M NaOH. When all solution A has been incorporated in the system, the bubbling and stirring are continued for 4 hours to guarantee an aging of the system which allows the growth of the LDH crystals. After this time period the solid is recovered by centrifugation (3600 rpm during 15 minutes) and it is washed several times with bidistilled water. The solid is later subjected to a controlled thermal treatment with a ramp heating of 26ºC/h until reaching 150ºC, the temperature at which the system is maintained for a further 3 hours. The solid obtained is subjected to a grinding process for its physicochemical characterization by X-ray diffraction techniques, Fourier transform infrared spectroscopy, differential thermal and thermogravimetric analysis, specific surface measurements and porosity, elemental chemical analysis, high resolution nuclear magnetic resonance of ²⁹Si, electronic scanning microscopy and electronic transmission microscopy. The LDH formation yields with respect to the theoretical value are close to 100% for all proportions tested. The X-ray diffraction technique makes it possible to confirm the formation of LDH (diffraction peaks at 0.774, 0.386, 0.261 and 0.191 nm). The results of nuclear magnetic resonance of ²⁹Si show the disappearance of the Q² signal (-85.0 ppm) assigned to the presence of silanol groups localized on the external surface of the sepiolite fibres, developing a new Q³ signal (96.7 ppm) which can be assigned to the formation of a new Si-O bond by condensation of superficial silanols of the sepiolite with hydroxyl groups of the LDH. Via electronic scanning and transmission microscopy it is possible to observe the growth of micro- and nano-particles of LDH on the sepiolite fibres whereto they are associated coating them.

### Example 3. - Preparation of composites which comprise the LDH of formula [Mg₂Al(OH)₆]Cl·xH₂O and palygorskite.

The same is done as in example 1, except that instead of sepiolite rheological grade palygorskite is used (supplied by the company Tolsa SA). Solution A has been, in this case, 0.453 g of AlCl₃.6H₂O and 0.763 g of MgCl₂.6H₂O in 500 ml of bidistilled water, to give a theoretical proportion of 0.4:1 LDH-palygorskite. The solid obtained once dry is subjected to a grinding process for its physicochemical characterization which indicates that the LDH has been formed and which coats the external surface of the palygorskite particles.

### Example 4. - Preparation of composites which comprise the LDH of formula [Mg₂Al(OH)₆]Cl·xH₂O and smectites of various characteristics

The same is done as in example 3, except that, instead of palygorskite, smectites of different characteristics are used: 1) a montmorillonite from Wyoming, USA, standard of SWy-1 type supplied by the Clay Source Repository of the Clay Mineral Society of the United States; 2) a montmorillonite marketed by the company Southern Clay Products with the name Cloisite®; and 3) a synthetic hectorite known as Laponite® marketed by the company Laporte, in all cases to obtain a theoretical LDH:mectite proportion by weight of 0.4:1. The solids obtained are subjected to a grinding process for their physicochemical characterization which indicates that the LDH has been formed and which coats the external surface of the particles of the smectites indicated, especially in accordance with the X-ray diffraction data and with the electronic transmission and scanning microscopies.

### Example 5. - Preparation of composites which comprise the LDH of formula [Mg₂Al(OH)₆]Cl·xH₂O and vermiculite.

The same is done as in example 3, except that, instead of palygorskite, vermiculite is used from Benahavis (Malaga, Spain), screened in a < 200 mesh size. Solution A has been, in this case, 0.453 g of AlCl₃.6H₂O and 0.763 g of MgCl₂.6H₂O in 500 ml of bidistilled water, to give a theoretical proportion of 0.4:1 LDH-vermiculite. The solid obtained once dry is subjected to a grinding process for its physicochemical characterization which indicates that the LDH has been formed and which coats the external surface of the vermiculite particles.

### Example 6. - Use of composites of the LDH of formula [Mg₂Al(OH)₆]Cl·xH₂O and sepiolite as adsorbent of anionic species.

Composites of LDH of formula [Mg₂Al(OH)₆]Cl·xH₂O and sepiolite with different relative compositions LDH:sepiolite (0.1:1, 0.2:1, 0.3:1, 0.4:1, 0.5:1, 1:1) have been used as adsorbents of anionic species using the anionic dye Congo Red as model molecule. This compound has been chosen since, for different concentrations of the dye, a correlation can be found between light absorption in the visible region of the spectrum at 498 nm (wavelength of maximum absorption) and the concentration of dye in an aqueous solution if this is between 5 and 25 ppm (region where the Lamber-Beer law is complied with). To determine the absorption capacity of the anionic dye in different LDH-sepiolite composites, 50 mg of this is placed in contact with 50 ml of a solution of 350 ppm in Congo Red during 1 day and under constant stirring. Afterwards, the samples are filtered and the quantity of Congo Red contained in the resulting liquid is determined by UV-visible spectroscopy and compared with the initial concentration to establish the quantity absorbed in the composite.

It is verified that the absorption capacity of the anionic dye of LDH by the LDH-sepiolite composites is in the range of 110 to 125% with respect to the theoretical quantity corresponding exclusively to the pure LDH. This fact indicates that the materials have an additional adsorption capacity which must be related to the presence of sepiolite. The adsorption capacity of the dye reaches values of around of 0.40 g/g of composite.

### Example 7. - Use of composites of the LDH of formula [Mg₂Al(OH)₆]Cl·xH₂O and sepiolite as absorbent of cationic species.

Composites of LDH of formula [Mg₂Al(OH)₆]Cl·xH₂O and sepiolite of relative composition LDH:sepiolite 0.3:1 have been used as adsorbent of cationic species using the cationic dye Methylene Blue as model molecule. This compound has been chosen since, for different concentrations of the dye, a correlation can be found between light absorption in the visible region of the spectrum at 664 nm (wavelength of maximum absorption) and the concentration of dye in an aqueous solution if this is between 1 and 6 ppm (region where the Lamber-Beer law is complied with). To determine the absorption capacity of the anionic dye in different LDH-sepiolite composites 30 mg of this is placed in contact with 250 ml of a solution of 18.6 ppm in Methylene Blue during 1 day and under constant stirring. Afterwards, the samples are filtered and the quantity of Methylene Blue contained in the resulting liquid is determined by UV-visible spectroscopy and compared with the initial concentration to establish the quantity absorbed in the composite.

The adsorption capacity of the cationic dye is around 5 mg/g of composite after washes in distilled water.

### Example 8. - Use of composites of the LDH of formula [Mg₂Al(OH)₆]Cl·xH₂O and sepiolite as adsorbent of anionic and cationic species

Composites of LDH of formula [Mg₂Al(OH)₆]Cl·xH₂O and sepiolite of relative composition LDHaepiolite: 0.1:1, 0.3:1, 1:1 have been used as adsorbents of both anionic and cationic species. In this study, the anionic dye Congo Red has first been absorbed as specified in Example 6 and then the materials are placed in contact with the cationic dye Methylene Blue and the same is done as in Example 7. Using UV-visible spectroscopy of the dye solutions and CHN elemental chemical analysis, the adsorption capacity of anionic and cationic species is established in the solids. These composites have the capacity of absorbing both types of dyes due to the contribution of each component, i.e. sepiolite and LDH, reaching values of 0.40 g Congo Red and 15 mg Methylene Blue per gram of composite.

### Example 9. - Use of composites of the LDH of formula [Zn₂Al(OH)₆]Cl·xH₂O and sepiolite as precursors of supported metal oxides.

The composite [Zn₂Al(OH)₆]Cl·xH₂O and sepiolite of composition 0.3:1 by weight prepared according to Example 2, has been used as precursor of metal oxides supported on sepiolite. For this purpose, the composite has been calcined in air at 600ºC during 3 hours (heating until the calcinations temperature with ramp of 10°-C/min). X-ray diffraction is used to check the formation of ZnO (ICSD #154486). Electronic scanning and transmission microscopes are used to check the formation of oxide particles on the sepiolite fibres.

## Claims

1. Micro- or nano-structured composite, **characterized in that** it comprises a layered double hydroxide of hydrotalcite type and a silicate of the clay family.

2. Composite according to claim 1, **characterized in that** the silicate of the clay family has a fibrous morphology.

3. Composite according to claim 2, **characterized in that** the silicate with fibrous morphology is sepiolite.

4. Composite according to claim 2, **characterized in that** the silicate with fibrous morphology is palygorskite.

5. Composite according to claim 1, **characterized in that** the silicate of the clay family has a layered structure.

6. Composite according to claim 5, **characterized in that** the silicate of layered structure is a natural silicate.

7. Composite according to claim 6, **characterized in that** the layered natural silicate is a smectite clay.

8. Composite according to claim 7, **characterized in that** the smectite clay is montmorillonite.

9. Composite according to claim 5, **characterized in that** the layered silicate is a synthetic silicate.

10. Composite according to claim 9, **characterized in that** the layered synthetic silicate is synthetic hectorite.

11. Process for preparation of the materials described in preceding claims, **characterized in that** it comprises the in situ formation of the layered double hydroxide in the presence of a clay.

12. Process according to claim 11, **characterized in that** the formation of the layered double hydroxide is carried out by co-precipitation.

13. Process according to claim 11, **characterized in that** the preparation of the composite involves a hydrothermal treatment.

14. Process according to claim 11, **characterized in that** the preparation of the composite involves a conventional thermal treatment.

15. Process according to claim 11, **characterized in that** the preparation of the composite involves a treatment by microwave irradiation.

16. Use of the composites described in claims 1-10 as adsorbent or absorbent.

17. Use of the composites according to claim 16 as gas adsorbent.

18. Use of the composites according to claim 16 as adsorbent of contaminants in aqueous media.

19. Use of the composites described in claims 1-10 as acid neutralizer.

20. Use of the composites described in claims 1-10 as ion exchanger.

21. Use of the composites described in claims 1-10 as active principle in medical and biological applications.

22. Use of the composites described in claims 1-10 as antacid agent.

23. Use of the composites described in claims 1-10 as fillers of PVC and other polymers.

24. Use of the composites described in claims 1-10 as precursors of metal oxides.

25. Use of the composites according to the description in claims 1-10 as corrosion protector agents.

26. Use of the composites according to claim 24 as precursors of catalysts.

27. Use of the composites according to claim 24 as precursors of adsorbents and absorbents.
